(19) Europäisches Patentamt
European Patent Office
Office européen des brevets

(11) **EP 3 906 402 B1**

(12) **FASCICULE DE BREVET EUROPEEN**

(45) Date de publication et mention
de la délivrance du brevet:
**07.12.2022 Bulletin 2022/49**

(21) Numéro de dépôt: **19850815.2**

(22) Date de dépôt: **28.12.2019**

(51) Classification Internationale des Brevets (IPC):
**G01N 21/552** *(2014.01)* **G01N 21/77** *(2006.01)*

(52) Classification Coopérative des Brevets (CPC):
**G01N 21/553; G01N 33/0031; G01N 33/0073;**
G01N 2021/7793

(86) Numéro de dépôt international:
**PCT/FR2019/053312**

(87) Numéro de publication internationale:
**WO 2020/141281 (09.07.2020 Gazette 2020/28)**

(54) **PROCEDE DE CARACTERISATION DE COMPOSES CIBLES**

VERFAHREN ZUR CHARAKTERISIERUNG VON ZIEL-VERBINDUNGEN

METHOD FOR CHARACTERISING TARGET COMPOUNDS

(84) Etats contractants désignés:
**AL AT BE BG CH CY CZ DE DK EE ES FI FR GB
GR HR HU IE IS IT LI LT LU LV MC MK MT NL NO
PL PT RO RS SE SI SK SM TR**

(30) Priorité: **31.12.2018 FR 1874420**

(43) Date de publication de la demande:
**10.11.2021 Bulletin 2021/45**

(73) Titulaire: **Aryballe**
**38000 Grenoble (FR)**

(72) Inventeurs:
• **CARITU, Yanis**
**38000 GRENOBLE (FR)**
• **ARKHIPOV, Kirill**
**38000 Grenoble Cedex 09 (FR)**

(74) Mandataire: **INNOV-GROUP**
**310, avenue Berthelot**
**69372 Lyon Cedex 08 (FR)**

(56) Documents cités:
**EP-A1- 1 645 880 WO-A1-03/081245**

• **TRINCAVELLI M ET AL: "Odour classification system for continuous monitoring applications", SENSORS AND ACTUATORS B: CHEMICAL, ELSEVIER BV, NL, vol. 139, no. 2, 20 mars 2009 (2009-03-20) , pages 265-273, XP026138567, ISSN: 0925-4005, DOI: 10.1016/J.SNB.2009.03.018 [extrait le 2009-03-20]**
• **PEARCE T C ED - LUCAS PHILIPPE ET AL: "Computational parallels between the biological olfactory pathway and its analogue 'The Electronic Nose': Part II. Sensor-based machine olfaction", BIOSYSTEMS, NORTH-HOLLAND, AMSTERDAM, NL, vol. 41, 1997, pages 69-90, XP007916532, ISSN: 0303-2647**
• **HAN FAN ET AL: "A cluster analysis approach based on exploiting density peaks for gas discrimination with electronic noses in open environments", SENSORS AND ACTUATORS B: CHEMICAL, vol. 259, 2 décembre 2017 (2017-12-02), pages 183-203, XP055630809, NL ISSN: 0925-4005, DOI: 10.1016/j.snb.2017.10.063**
• **SOPHIE BRENET ET AL: "Highly-Selective Optoelectronic Nose Based on Surface Plasmon Resonance Imaging for Sensing Volatile Organic Compounds", ANALYTICAL CHEMISTRY, vol. 90, no. 16, 19 juillet 2018 (2018-07-19), pages 9879-9887, XP055630144, US ISSN: 0003-2700, DOI: 10.1021/acs.analchem.8b02036 cité dans la demande**

**Description**

**DOMAINE TECHNIQUE**

Le domaine de l'invention est celui de la caractérisation de composés cibles présents dans un échantillon fluide, de préférence par un nez électronique utilisant une technologie d'imagerie par résonance plasmonique de surface.

**ÉTAT DE LA TECHNIQUE ANTÉRIEURE**

La capacité à caractériser et à analyser des composés cibles contenus dans des échantillons fluides, par exemple des molécules odorantes ou des composés organiques volatils, est une problématique de plus en plus importante dans différents domaines, notamment dans ceux de la santé, des senteurs dans l'industrie de la parfumerie, de l'industrie agroalimentaire, du confort olfactif dans les endroits confinés publics ou privés (automobile, hôtellerie, lieux partagés...), etc...

Différentes approches de caractérisation existent, qui se distinguent entre elles notamment par la nécessité ou non d'avoir à « marquer » au préalable les composés cibles ou les récepteurs par un agent de révélation. A la différence par exemple de la détection par fluorescence qui nécessite d'avoir recours à de tels marqueurs, la détection par résonance plasmonique de surface (SPR pour *Surface Plasmon Resonance,* en anglais) est une technique dite sans marqueur (*label free,* en anglais).

Elle peut être mise en œuvre par un nez électronique, notamment par imagerie SPR, lorsque les composés cibles sont contenus dans un échantillon gazeux ou liquide. La technique de caractérisation par résonance plasmonique de surface permet de mesurer en temps réel un signal optique représentatif des interactions d'adsorption et de désorption entre des composés cibles et des récepteurs disposés sur des sites sensibles du nez électronique. Dans la mesure où on ne connaît pas *a priori* l'affinité chimique ou physique d'interaction des composés cibles avec les récepteurs, la caractérisation des composés cibles consiste alors à fournir un motif d'interaction obtenu à partir des signaux optiques mesurés pour les sites sensibles. L'adsorption et la désorption sur une surface préparée (ou « fonctionnalisée ») bénéficiant de caractéristiques d'adsorption différenciées permettent de rendre compte des molécules présentes dans le gaz qui ont été accrochées par la surface. L'interaction des photons incidents avec le nuage électronique changeant de la surface crée un transfert d'énergie et met à jour les motifs selon l'excitation gazeuse.

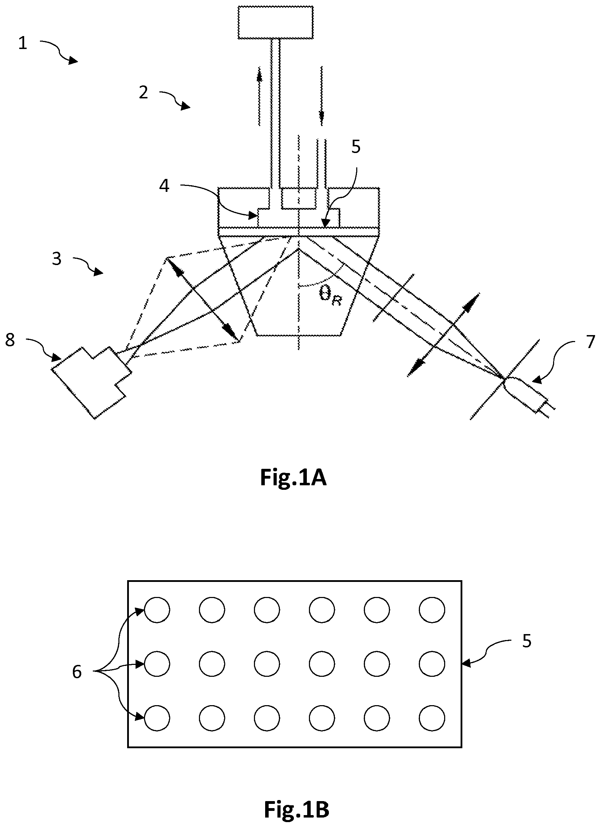

A ce titre, les figures 1A et 1B illustrent un exemple de nez électronique tel que décrit dans la demande WO2018/158458. Le nez électronique 1 comporte, d'une manière générale, un dispositif d'alimentation fluidique 2, un dispositif de mesure optique 3 par imagerie SPR, et une unité de traitement (non représentée).

Le dispositif de mesure optique 3 comporte une chambre de mesure 4 destinée à recevoir l'échantillon gazeux, dans laquelle se situe un support de mesure 5 sur lequel est située une matrice de sites sensibles 6. Le support de mesure 5 est formé d'une couche métallique sur laquelle sont fixés différents récepteurs adaptés à interagir avec les composés cibles, les différents récepteurs étant disposés de manière à former des sites sensibles 6 distincts les uns des autres. Ces récepteurs sont alors situés à l'interface entre la couche métallique et un milieu diélectrique, ici un milieu gazeux.

Il comporte en outre une source lumineuse 7 d'un rayonnement d'excitation et un capteur d'image 8. La source lumineuse 7 est adaptée à émettre un rayonnement lumineux d'excitation en direction du support de mesure 5, suivant un angle de travail $\theta_R$ permettant d'y générer des plasmons de surface. La partie réfléchie du rayonnement lumineux d'excitation est ensuite détectée par le capteur d'image 8. L'intensité optique du rayonnement réfléchi dépend localement de l'indice de réfraction du support de mesure 5, qui dépend lui-même des plasmons de surface générés et de la quantité de matière située au niveau de chaque site sensible 6, cette quantité de matière variant au cours du temps au grès des interactions entre les composés sensibles et les récepteurs.

L'unité de traitement du nez électronique est adaptée à analyser les « sensorgrammes », c'est-à-dire l'évolution temporelle de l'intensité optique du rayonnement réfléchi et mesuré par le capteur d'image 8, pour chaque site sensible 6, dans le but d'en extraire les informations cinétiques d'interaction (adsorption et désorption) des composés cibles avec les récepteurs des sites sensibles 6.

Enfin, le dispositif d'alimentation fluidique 2 est adapté à introduire les composés cibles dans la chambre de mesure 4 dans des conditions permettant de permettre l'analyse des sensorgrammes et donc la caractérisation des composés cibles.

A ce titre, l'article de Brenet et al. intitulé Highly-Selective Optoelectronic Nose based on Surface Plasmon Resonance Imaging for Sensing Gas Phase Volatile Organic Compounds, Anal. Chem. 2018, 90, 16, 9879-9887, décrit un procédé de caractérisation d'un échantillon gazeux au moyen d'un nez électronique de type imagerie SPR.

Le procédé de caractérisation consiste à alimenter la chambre de mesure en un échantillon gazeux de telle manière que la cinétique d'interaction entre les composés cibles et les récepteurs présente un régime stationnaire d'équilibre.

Plus précisément, l'étape d'alimentation fluidique comporte une première phase dite initiale, dans laquelle l'échantillon gazeux est formé du seul gaz porteur, sans composés cibles ; d'une deuxième phase dite d'injection, dans laquelle l'échantillon gazeux est formé du gaz porteur et des composés cibles ; et d'une troisième phase dite de dissociation, dans laquelle les composés cibles sont évacués de la chambre de mesure. La phase initiale permet d'acquérir un signal optique de référence destiné à être ensuite soustrait au signal optique de mesure acquis lors de la phase d'injection. Cette phase d'injection est réalisée, via le dispositif d'alimentation fluidique, de sorte que les sensorgrammes mettent en évidence la présence d'un régime transitoire d'assimilation suivi d'un régime stationnaire d'équilibre. Lorsque ce régime stationnaire d'équilibre est atteint, la caractérisation des composés cibles par l'unité de traitement est alors possible.

L'article de TRINCAVELLI M ET AL : "Odour classification system for continuous monitoring applications", SENSORS AND ACTUATORS B: CHEMICAL, ELSEVIER BV, NL, vol. 139, no. 2, 20 mars 2009 (2009-03-20), pages 265-273, décrit un procédé de caractérisation de composés cibles selon le préambule de la revendication 1.

L'article de PEARCE T C : "Computational parallels between the biological olfactory pathway and its analogue 'The Electronic Nose': Part II. Sensor-based machine olfaction", BIOSYSTEMS, NORTH-HOLLAND, AMSTERDAM, NL, vol. 41, 1997, pages 69-90, et l'article de HAN FAN ET AL : "A cluster analysis approach based on exploiting density peaks for gas discrimination with electronic noses in open environments", SENSORS AND ACTUATORS B: CHEMICAL, vol. 259, 2 décembre 2017 (2017-12-02), pages 183-203, décrivent d'autres procédés liés aux systèmes de nez électroniques.

Il existe cependant un besoin de disposer d'un procédé de caractérisation permettant d'obtenir la caractérisation des composés cibles de manière plus simple et plus rapide, que le régime stationnaire d'équilibre adsorption/désorption soit ou non présent entre les composés cibles et les récepteurs.

**EXPOSÉ DE L'INVENTION**

L'invention a pour objectif de remédier au moins en partie aux inconvénients de l'art antérieur, et plus particulièrement de proposer un procédé de caractérisation de composés cibles qui soit plus simple et rapide, sans qu'il soit nécessaire que les conditions opératoires d'alimentation fluidique et la structure du dispositif d'alimentation fluidique soient prévues pour obtenir le régime stationnaire d'équilibre adsorption/désorption au sein de la chambre de mesure. Le procédé de caractérisation peut être mis en œuvre par un nez électronique à technologie SPR, et de préférence à imagerie SPR, ainsi que par d'autres technologies, comme par exemple celle des résonateurs électromécaniques de type NEMS ou MEMS.

Pour cela, l'objet de l'invention est un procédé de caractérisation de composés cibles, par un système d'analyse comportant une chambre de mesure destinée à recevoir les composés cibles contenus dans un échantillon fluide, dans laquelle est située une pluralité de sites sensibles distincts comportant chacun des récepteurs aptes à interagir avec les composés cibles, le procédé comportant les étapes suivantes :

o alimentation fluidique d'un échantillon fluide dans la chambre de mesure, comportant une phase d'injection $P_2$ dans laquelle l'échantillon fluide est formé d'un fluide porteur et desdits composés cibles ;
o détermination, lors de l'étape d'alimentation, à un instant de mesure $t_i$, pour chaque site sensible, d'un signal de mesure $S_k(t_i)$ représentatif des interactions entre les composés cibles et les récepteurs, k étant le rang du site sensible considéré, de manière à obtenir un vecteur dit de mesure $S(t_i)$ formé des signaux de mesure $S_k(t_i)$ acquis à l'instant de mesure $t_i$ ;

Selon l'invention, le procédé comporte en outre les étapes suivantes :

o calcul, à l'instant de mesure $t_i$, d'un vecteur normé $Sn(t_i)$ à partir du vecteur de mesure $S(t_i)$ à l'instant de mesure $t_i$ et d'une norme $\|S(t_i)\|$ calculée à partir du vecteur de mesure $S(t_i)$ à l'instant de mesure $t_i$ ;
o réitération des étapes de détermination des signaux de mesure et de calcul du vecteur normé, en incrémentant l'instant de mesure, jusqu'à l'atteinte d'un critère de stabilité, de manière à obtenir une caractérisation des composés cibles, à partir du vecteur normé $Sn'(t_i)$ à l'instant de mesure $t_i$.

Certains aspects préférés mais non limitatifs de ce procédé de caractérisation sont les suivants.

L'étape d'alimentation fluidique peut comporter, au préalable de la phase d'injection P2, une phase initiale P1 dans laquelle l'échantillon fluide est formé du fluide porteur sans lesdits composés cibles. L'étape de détermination du signal de mesure $S_k(t_i)$ peut comporter un calcul d'un vecteur dit utile $Su(t_i)$ à l'instant de mesure $t_i$, par soustraction au vecteur de mesure $S(t_i)$ d'un vecteur dit de référence $S(\Delta t_{ref})$ déterminé lors de la phase initiale P1 pour une période de mesure $\Delta t_{ref}$ prédéterminée. Le vecteur normé $Sn(t_i)$ peut être calculé à partir du vecteur utile $Su(t_i)$.

L'étape de détermination du signal de mesure $S_k(t_i)$ peut comporter un calcul d'un vecteur dit corrigé $Sc(t_i)$ à partir du vecteur de mesure $S(t_i)$ par application d'un filtre passe-bas ou d'un cumul des valeurs du vecteur de mesure $S(t_i)$ aux instants de mesure précédents.

Le critère de stabilité peut comporter une comparaison, à l'instant de mesure $t_i$, d'un paramètre dit de stabilité $P_{st}(t_i)$ calculé à partir des coordonnées $Sn_k(t_i)$ du vecteur normé $Sn(t_i)$ sur une fenêtre glissante $t_i-T_{st}$, vis-à-vis d'une valeur seuil déterminée $P_{st,th}$.

Le paramètre de stabilité $P_{st}(t_i)$ peut être le maximum parmi des variances calculées à l'instant de mesure ti pour les coordonnées $Sn_k(t_i-T_{st})$ du vecteur normé $Sn$ sur une fenêtre glissante $t_i-T_{st}$.

Le critère de stabilité peut comporter une comparaison, à l'instant de mesure $t_i$, d'un paramètre dit d'injection $P_{inj}(t_i)$ calculé à partir des coordonnées $S_k(t_i)$ du vecteur de mesure $S(t_i)$ sur une fenêtre glissante $t_i-T_{inj}$, vis-à-vis d'une valeur seuil déterminée $P_{inj,th}$.

Le paramètre d'injection $P_{inj}(t_i)$ peut être le maximum parmi des variances calculées à l'instant de mesure $t_i$ pour les coordonnées $S_k(t_i-T_{inj})$ du vecteur de mesure **S** sur une fenêtre glissante $t_i-T_{inj}$.

La norme $\|S(ti)\|$ est de préférence la norme euclidienne.

L'étape de caractérisation peut comporter la fourniture d'au moins un paramètre caractéristique d'une évolution temporelle de la norme euclidienne du vecteur normé **Sn** lors de la phase d'injection P2.

L'étape de caractérisation peut comporter un calcul d'une intégrale, sur la durée de la phase d'injection P2, de la norme euclidienne du vecteur normé **Sn.**

Le système d'analyse peut être un nez électronique à imagerie par résonance plasmonique de surface, ou peut être un système d'analyse comportant une pluralité de résonateurs électromécaniques distincts formant chacun un site sensible.

## BRÈVE DESCRIPTION DES DESSINS

D'autres aspects, buts, avantages et caractéristiques de l'invention apparaîtront mieux à la lecture de la description détaillée suivante de formes de réalisation préférées de celle-ci, donnée à titre d'exemple non limitatif, et faite en référence aux dessins annexés sur lesquels :

la figure 1A et 1B, déjà décrites, sont des vues schématiques et partielles, en coupe (fig.1A) et en vue de dessus (fig.1B), d'un nez électronique selon un exemple de l'art antérieur et des sites sensibles du support de mesure ;

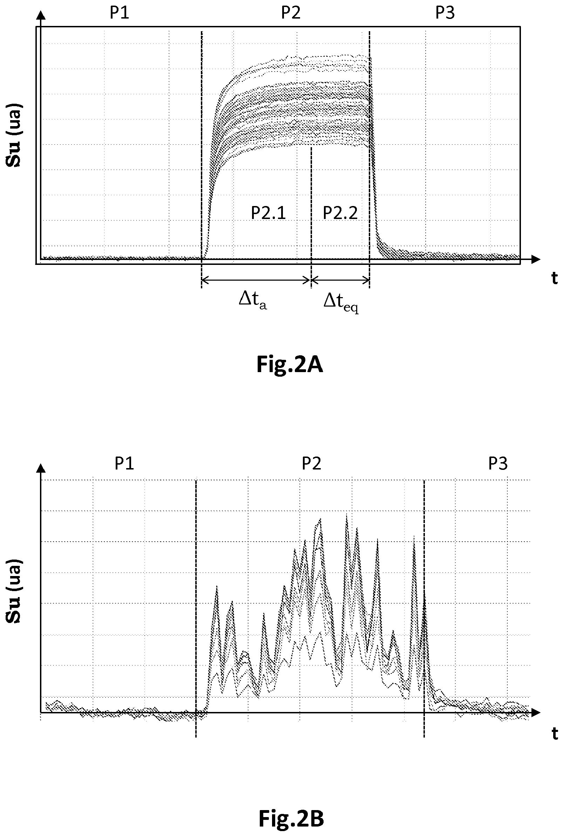

les figures 2A et 2B sont des exemples de sensorgrammes $Su_k(t_i)$ mesurés par le nez électronique, c'est-à-dire des exemples d'évolution temporelle de l'intensité optique du rayonnement lumineux réfléchi par différents sites sensibles et mesurée par le capteur d'image du nez électronique, dans le cas de profils dits conventionnels (fig.2A) et dans le cas de profils dits dégradés (fig.2B) ;

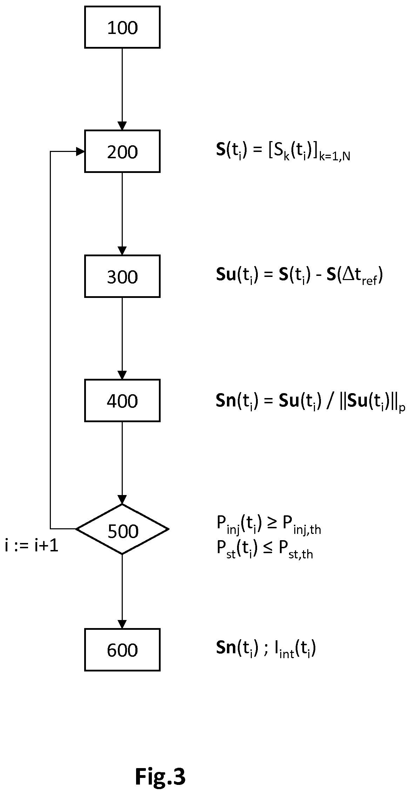

la figure 3 est un organigramme illustrant les différentes étapes d'un procédé de caractérisation selon un mode de réalisation ;

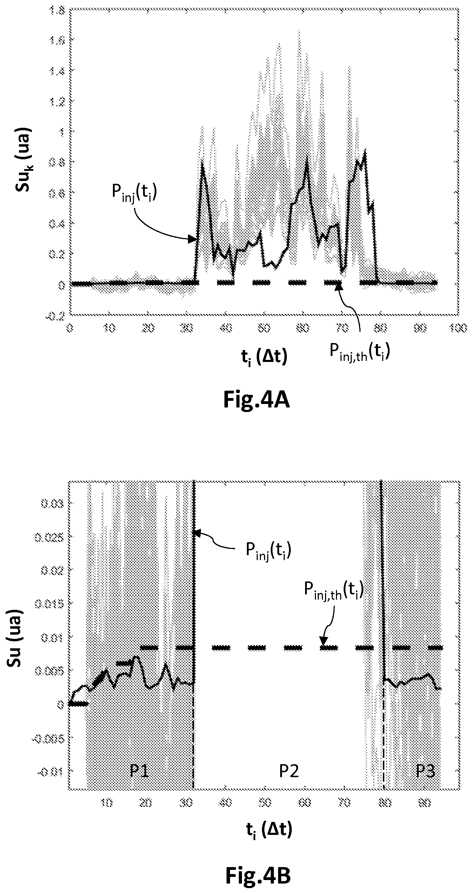

la figure 4A illustre l'évolution temporelle de sensorgrammes $Su_k(t_i)$ à profil dégradé, ainsi que l'évolution temporelle (trait gras continu) du paramètre d'injection $P_{inj}(t_i)$ et celle (trait gras en pointillés) de la valeur seuil $P_{inj,th}(t_i)$ de celui-ci, et la figure 4B illustre une vue partielle et en détail de la fig.4A, montrant plus précisément les variations temporelles de $P_{inj}(t_i)$ et de $P_{inj,th}(t_i)$, et permettant d'identifier la phase d'injection P2 des composés cibles lors de l'étape d'alimentation fluidique ;

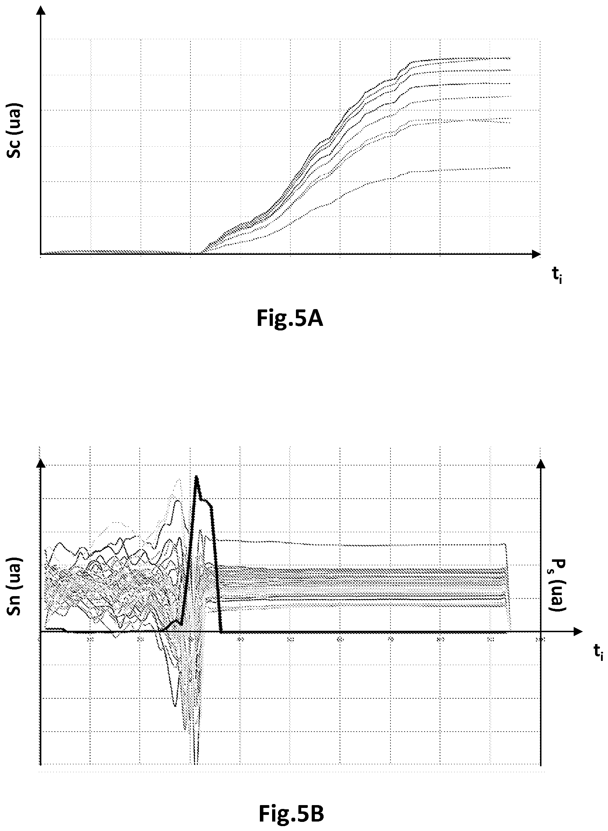

les figures 5A et 5B illustrent un exemple d'évolution temporelle (fig.5A) de signaux corrigés $Sc_k(t_i)$ obtenus par cumul des sensorgrammes $Su_k(t_i)$ à profil dégradé identiques ou similaires à ceux illustrés sur la fig.2B, ainsi qu'un exemple d'évolution temporelle (fig.5B) de signaux normés $Sn_k(t_i)$ ainsi que celle du paramètre de stabilité $P_s(t_i)$ correspondant ;

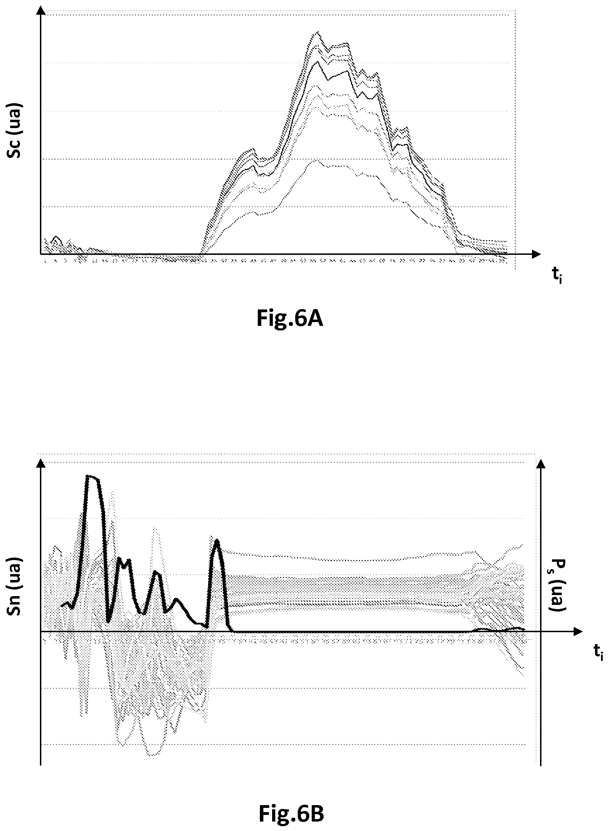

les figures 6A et 6B illustrent un exemple d'évolution temporelle (fig.6A) de signaux corrigés $Sc_k(t_i)$ obtenus par filtrage passe-bas des sensorgrammes $Su_k(t_i)$ à profil dégradé identiques ou similaires à ceux illustrés sur la fig.2B, ainsi qu'un exemple d'évolution temporelle (fig.6B) de signaux normés $Sn_k(t_i)$ ainsi que celle du paramètre de stabilité $P_s(t_i)$ correspondant ;

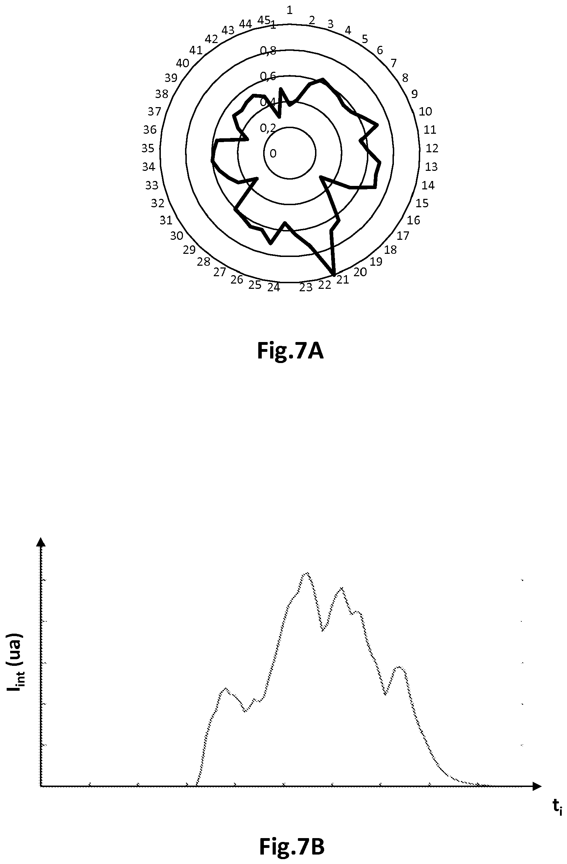

la figure 7A illustre un exemple de motif d'interaction obtenu par le procédé de caractérisation selon un mode de réalisation, dans le cas où les sensorgrammes présentent un profil dégradé, et la figure 7B illustre un exemple d'évolution temporelle d'une intensité d'interaction instantanée $I_{int}(t_i)$ associée aux sensorgrammes identiques ou similaires à ceux illustrés sur la fig.2B.

## EXPOSÉ DÉTAILLÉ DE MODES DE RÉALISATION PARTICULIERS

Sur les figures et dans la suite de la description, les mêmes références représentent les éléments identiques ou similaires. De plus, les différents éléments ne sont pas représentés à l'échelle de manière à privilégier la clarté des figures. Par ailleurs, les différents modes de réalisation et variantes ne sont pas exclusifs les uns des autres et peuvent être combinés entre eux. Sauf indication contraire, les termes « sensiblement », « environ », « de l'ordre de » signifient à 10% près, et de préférence à 5% près.

L'invention porte sur la caractérisation de composés cibles contenus dans un échantillon fluide au moyen d'un système d'analyse comportant un dispositif de mesure, un dispositif d'alimentation fluidique et une unité de traitement. Comme détaillé plus loin, le dispositif de mesure comporte une chambre de mesure adaptée à recevoir un échantillon fluide (gazeux ou liquide) comportant les composés cibles, dans laquelle est située une pluralité de sites sensibles distincts, ayant chacun au moins un récepteur adapté à interagir par adsorption/désorption avec les composés cibles.

Dans la suite de la description, le système d'analyse est un nez électronique à imagerie par résonance plasmonique de surface (SPR). Cependant, d'autres technologies de caractérisation peuvent être utilisées. A ce titre, le système d'analyse peut, en variante au nez électronique, comporter des dispositifs MEMS (*Micro-Electro-Mechanical System,* en anglais) ou NEMS (*Nano-Electro-Mechanical System,* en anglais) de type résonateur. Ce type de technologie est connu de l'homme du métier, et un exemple d'un tel système d'analyse est décrit dans le document EP3184485. Le système d'analyse comporte une pluralité de résonateurs électromécaniques distincts les uns des autres, une surface de chaque résonateur étant fonctionnalisée par la présence de récepteurs, et forme ainsi un site sensible. De manière connue, les interactions entre les composés cibles et les récepteurs d'un site sensible provoquent une modification de la fréquence de résonance du résonateur électromécanique. De manière similaire à la technologie SPR du nez électronique, la mesure de la variation de la fréquence de résonance permet ainsi d'obtenir un signal de mesure $S_k(t_i)$ représentatif des interactions entre les composés cibles et les récepteurs du site sensible de rang k considéré. La mesure de la variation de la fréquence de résonance peut être effectuée via une mesure de type piézorésistif, capacitif, ou autre.

D'une manière générale, par caractérisation, on entend l'obtention d'informations représentatives des interactions des composés cibles contenus dans l'échantillon fluide avec des récepteurs de différents sites sensibles du système d'analyse. Les interactions en question ici sont des évènements d'adsorption et/ou de désorption des composés cibles avec des récepteurs. Ces informations forment ainsi un motif d'interaction, autrement dit une « signature » des composés cibles, ce motif pouvant être représenté par exemple sous forme d'histogramme ou d'un diagramme en radar. Plus précisément, dans le cas où le système d'analyse comporte N sites sensibles distincts, le motif d'interaction est formé par les N informations représentatives, celles-ci étant formées d'une valeur corrélée à l'intensité d'un signal optique de mesure provenant du site sensible considéré.

D'une manière générale, les composés cibles sont des éléments destinés à être caractérisés par le nez électronique, et contenus dans un échantillon fluide. Ils peuvent être, à titre illustratif, des bactéries, virus, protéines, lipides, molécules organiques volatiles, composés inorganiques, entre autres. Par ailleurs, les récepteurs sont des éléments fixés aux sites sensibles et qui présentent une capacité d'interaction avec les composés cibles, bien que les affinités chimique et/ou physique entre les composés sensibles et les récepteurs ne soient pas nécessairement connues. Les récepteurs des différents sites sensibles présentent de préférence des propriétés physico-chimiques différentes, qui impactent leur capacité à interagir avec les composés cibles. Il peut s'agir, à titre d'exemples, des acides aminés, des peptides, des nucléotides, des polypeptides, des protéines, des polymères organiques, entre autres.

En référence aux fig.1A et 1B décrites succinctement plus haut, le nez électronique 1 est un système optoélectronique permettant de caractériser des composés cibles, par exemple des molécules odorantes, des composés organiques volatils ou autres, contenus dans un échantillon fluide introduit dans une chambre de mesure 4 du nez électronique. Le nez électronique 1 représenté sur ces figures présente ici les caractéristiques de la configuration dite de Kretschmann, connue de l'homme du métier, sans que l'invention ne soit toutefois limitée à cette configuration. L'échantillon fluide peut être un échantillon liquide ou gazeux. Dans la suite de la description, il s'agit d'un échantillon gazeux.

Le nez électronique 1 comporte, située dans une chambre de mesure 4 destinée à recevoir l'échantillon gazeux à analyser, une pluralité de sites sensibles 6 distincts les uns des autres, formés chacun des récepteurs aptes à interagir avec les composés cibles à étudier et donc aptes à interagir de manière différenciée avec l'échantillon. Les sites sensibles 6 sont distincts les uns des autres, dans le sens où ils comportent des récepteurs différents, en termes d'affinité chimique ou physique vis-à-vis des composés cibles à analyser, et sont donc destinés à fournir une information d'interaction différente d'un site sensible 6 à l'autre. Les sites sensibles 6 sont des zones distinctes d'un support de mesure 5, et peuvent être accolés ou espacés les unes des autres. Le nez électronique 1 peut en outre comporter plusieurs sites sensibles 6 identiques, dans le but par exemple de détecter une éventuelle dérive de mesure ou l'identification d'un site sensible défectueux.

Le nez électronique comporte un dispositif de mesure optique 3 de type imagerie SPR, permettant de quantifier les interactions des composés cibles avec les récepteurs, pour chaque site sensible 6, ici en mesurant l'intensité d'un signal optique associé au site sensible 6 considéré, ce signal optique étant une partie ici réfléchie d'un rayonnement lumineux d'excitation émis par une source lumineuse. L'intensité du signal optique mesuré est directement corrélée aux interactions entre les composés cibles avec les récepteurs.

Dans le cadre de la mesure par imagerie SPR, le dispositif de mesure optique 3 est adapté à acquérir en temps réel un rayonnement lumineux provenant de l'ensemble des sites sensibles 6. Ainsi, les signaux optiques émis par la pluralité de sites sensibles 6 sont mesurés ensemble et en temps réel, sous la forme d'une image acquise par le même capteur optique 8.

Ainsi, le dispositif de mesure optique 3 comporte une source lumineuse 7 adaptée à transmettre un rayonnement lumineux dit d'excitation en direction des sites sensibles 6, et à générer des plasmons de surface au niveau du support de mesure 5. La source lumineuse 7 peut être formée d'une diode électroluminescente, dont le spectre d'émission présente un pic d'émission centré sur une longueur d'onde centrale $\lambda_c$. Différents éléments optiques (lentilles, polariseur...) peuvent être disposés entre la source lumineuse 7 et le support de mesure 5.

Le dispositif de mesure optique 3 comporte en outre un capteur d'image 8, c'est-à-dire un capteur optique matriciel adapté à collecter une image du rayonnement lumineux provenant des sites sensibles en réponse au rayonnement lumineux d'excitation. Le capteur d'image 8 est un photodétecteur matriciel, par exemple un capteur CMOS ou CCD. Il comporte donc une matrice de pixels dont la résolution spatiale est telle que, de préférence, plusieurs pixels acquièrent une partie du rayonnement lumineux réfléchi associée à un même site sensible 6.

L'unité de traitement (non représentée) permet la mise en œuvre des opérations de traitement décrites par la suite dans le cadre du procédé de caractérisation. Elle peut comporter au moins un microprocesseur et au moins une mémoire. Elle est connectée au dispositif de mesure optique 3, et plus précisément au capteur d'image 8. Elle comporte un processeur programmable apte à exécuter des instructions enregistrées sur un support d'enregistrement d'informations. Elle comporte en outre au moins une mémoire contenant les instructions nécessaires à la mise en œuvre du procédé de caractérisation. La mémoire est également adaptée à stocker les informations calculées à chaque instant de mesure.

Comme décrit plus loin, l'unité de traitement est notamment adaptée à stocker et à traiter une pluralité d'images dites élémentaires acquises à une fréquence d'échantillonnage $f_e$ donnée, sur une durée de mesure $\Delta t$, afin de déterminer un signal optique de mesure $S_k(t_i)$, à l'instant courant $t_i$, associé au site sensible de rang k.

Le dispositif d'alimentation fluidique 2 est adapté à alimenter la chambre de mesure 4 en échantillons gazeux, celui étant formé d'un gaz porteur, avec ou sans composés cibles. Comme mentionné précédemment, les échantillons fluides peuvent, en variante, être en phase liquide (liquide porteur, avec ou sans composés cibles). Pour cela, il comporte un réservoir de gaz porteur, et une source de composés cibles. Il peut comporter plusieurs lignes fluidiques, reliées à l'entrée de la chambre de mesure 4, et peut comporter des vannes et régulateurs d'écoulement massique. Il permet ainsi d'alimenter la chambre de mesure 4 en au moins un gaz porteur sans composés cibles, par exemple pour la phase initiale et la phase de dissociation, et permet d'injecter les composés cibles au cours de la phase d'injection. Il peut être adapté à assurer que la concentration des composés cibles dans la chambre de mesure reste constante au cours du temps, ou non.

La figure 2A illustre un exemple de sensorgrammes $Su_k(t)$ associés chacun à chaque site sensible du nez électronique, dans le cadre d'un procédé de caractérisation dans lequel les sensorgrammes présentent chacun un profil dit conventionnel, c'est-à-dire que, comme explicité plus loin, ils mettent en évidence la présence d'un régime stationnaire d'équilibre entre les composés cibles et les récepteurs.

Un sensorgramme correspond à l'évolution temporelle d'un signal représentatif des interactions entre les composés cibles et les récepteurs d'un site sensible. Dans cet exemple, il s'agit de l'intensité d'un signal optique dit utile $Su_k(t)$ associé au site sensible de rang k, et plus précisément ici de la variation de réflectivité $\Delta R$ correspondant à la modification de l'indice de réfraction du site sensible de rang k considéré, liée aux interactions d'adsorption et de désorption des composés cibles avec les récepteurs du site sensible.

De manière connue, un sensorgramme à profil conventionnel présente une phase initiale P1, une phase d'injection des composés cibles P2, puis une phase de dissociation P3. L'intensité du signal du sensorgramme est proportionnelle au nombre de récepteurs du site sensible considéré.

La phase initiale P1 correspond à l'introduction dans la chambre de mesure du seul fluide porteur, sans composés cibles. Les sensorgrammes renvoient ainsi un signal de référence qui caractérise l'environnement de la mesure. Ce signal de référence est destiné à être ultérieurement soustrait du signal de mesure pour obtenir ainsi un signal utile représentatif des interactions des composés cibles.

La phase d'injection P2 des composés cibles correspond à l'introduction dans la chambre de mesure d'un échantillon fluide formé du fluide porteur et des composés cibles. Cette phase comporte un régime transitoire d'assimilation P2.1 suivi d'un régime stationnaire d'équilibre P2.2.

Le régime transitoire d'assimilation P2.1 correspond à l'augmentation progressive, de forme exponentielle (loi de Langmuir), des interactions entre les composés cibles et les récepteurs, à mesure que les composés cibles sont injectés dans la chambre de mesure. La croissance exponentielle des sensorgrammes dans le régime d'assimilation est due au fait qu'il y a, alors, bien plus d'événements d'adsorption que d'événements de désorption.

Notons à cet égard que l'interaction entre un composé cible A (*analyte* en anglais) et un récepteur L (*ligand,* en anglais) est un phénomène réversible caractérisé par une constante d'adsorption $k_a$ (en mol$^{-1}$.s$^{-1}$) du composé cible A au récepteur L pour former un composé cible/récepteur LA (pour *ligand-analyte,* en anglais), et par une constante de désorption $k_b$ (en s$^{-1}$) correspondant à la dissociation du composé LA. Le ratio $k_d/k_a$ forme la constante de dissociation d'équilibre $k_D$ (en mol) qui donne la valeur de la concentration $c_A$ de composés cibles A permettant de saturer 50% des récepteurs L.

Le régime stationnaire d'équilibre P2.2 est atteint lorsque la concentration $c_{LA}(t)$ en composés LA est stationnaire $dc_{LA}/dt=0$, c'est-à-dire lorsque le produit de la constante $k_a$ avec les concentrations de composés cibles $c_A(t)$ et de récepteurs $c_L(t)$ (nombre d'événements d'adsorption) est égal au produit de la constante $k_d$ avec la concentration $c_{LA}(t)$ de composés LA (nombre d'événements de désorption), autrement dit lorsque l'équation d'évolution suivante est vérifiée $dc_{LA}/dt = k_a \times c_A \times c_L - k_d \times c_{LA} = 0$. La valeur maximale stationnaire du signal de réponse est proportionnelle à la concentration $c_A(t)$ de composés cibles A. La saturation des récepteurs L du site sensible peut être atteinte lorsque la concentration $c_A$ en composés cibles A est suffisante.

La phase de dissociation P3 correspond à une étape d'évacuation des composés cibles présents dans la chambre de mesure, de sorte que la concentration en composés LA diminue, habituellement de manière exponentielle.

Il ressort de l'équation d'évolution indiquée ci-dessus que le régime stationnaire d'équilibre P2.2 requiert que la concentration $c_A(t)$ en composés cibles A reste constante dans la chambre de mesure au cours de l'étape d'injection P2. Le régime d'équilibre ne peut donc être atteint lorsque la concentration $c_A(t)$ en composés cibles A varie dans le temps. Cela nécessite donc que le dispositif d'alimentation fluidique du nez électronique soit en mesure de contrôler précisément la concentration $c_A(t)$ des composés cibles dans la chambre de mesure, et que le procédé de caractérisation comporte un protocole rigoureux de gestion fluidique des composés cibles ainsi que des conditions opératoires strictes et maîtrisées.

La figure 2B illustre un autre exemple de sensorgrammes $Su_k(t)$ associés aux sites sensibles du nez électronique. Ils se distinguent de ceux illustrés sur la fig.2A notamment en ce que les profils ne présentent pas de régime stationnaire d'équilibre P2.2, et sont alors dits dégradés.

Les sensorgrammes $Su_k(t)$ sont obtenus au cours d'une étape d'alimentation fluidique qui comporte également une phase initiale P1, une phase d'injection des composés cibles P2 et une phase de dissociation P3. Les phases initiale P1 et de dissociation P3 sont ici similaires à celles décrites précédemment. En revanche, lors de la phase d'injection P2, il apparaît que les sensorgrammes $Su_k(t)$ présentent de fortes variations en intensité, de sorte qu'il n'est alors pas possible d'identifier un quelconque régime transitoire d'assimilation P2.1 ni un régime stationnaire d'équilibre P2.2.

Ce type de profils dégradés de sensorgrammes peut être représentatif d'une situation dans laquelle la concentration $c_A(t)$ en composés cibles au sein de la chambre de mesure varie au cours du temps. Il ne peut alors être obtenu un régime stationnaire d'équilibre entre les cinétiques d'adsorption et de désorption des composés cibles avec les récepteurs. Ces sensorgrammes peuvent être obtenus au moyen d'un dispositif d'alimentation fluidique simplifié, lequel ne permet pas de contrôler la valeur de la concentration $c_A(t)$ au cours du temps, notamment lors d'une utilisation en conditions réelles, non contrôlées, du nez électronique où les composés cibles ne présentent alors pas une concentration $c_A(t)$ constante. A titre d'exemple, il peut ainsi s'agir de l'injection d'une odeur présente dans l'air libre et dont la concentration ne peut être contrôlée.

Le procédé de caractérisation selon l'invention permet de fournir une caractérisation des composés cibles à partir de sensorgrammes à profils de type conventionnel ou dégradé, c'est-à-dire que la concentration $c_A(t)$ des composés cibles soit constante ou non dans la chambre de mesure. Ce procédé de caractérisation présente en outre l'avantage de pouvoir fournir des informations supplémentaires relatives aux composés cibles, telles qu'une intensité d'interaction instantanée $I_{int}(t_i)$ représentative du peuplement instantané des composés cibles adsorbés par les récepteurs, ainsi qu'un niveau d'exposition total des récepteurs aux composés cibles sur la période de la phase d'injection P2. Le caractère « instantané » est ici relatif au temps d'intégration du capteur d'image, de sa période d'acquisition $\Delta t$, etc... et non pas aux temps caractéristiques effectifs des phénomènes physiques d'adsorption/désorption (bien plus rapides).

La figure 3 illustre un organigramme d'un procédé de caractérisation de composés cibles selon un mode de réalisation. L'échantillon fluide est ici un échantillon gazeux.

Lors d'une première étape 100, on active l'alimentation fluidique de la chambre de mesure du nez électronique en un échantillon gazeux. Cette étape comporte une première phase initiale P1, une phase d'injection des composés cibles P2, puis une phase de dissociation P3.

Dans cet exemple, le dispositif d'alimentation fluidique est adapté à injecter des composés cibles dans la chambre de mesure sans que la concentration $c_A(t)$ ne reste nécessairement constante. La concentration $c_A(t)$ peut rester constante, comme elle peut présenter des variations temporelles importantes. Quoi qu'il en soit, on suppose que la concentration $c_A(t)$ reste constamment supérieure à une concentration minimale correspondant à la limite de détection du nez électronique. Aussi, la phase d'injection ne présente pas nécessairement de régime stationnaire d'équilibre.

Les étapes suivantes de détermination des signaux de mesure et de traitement des données sont effectuées au cours de l'étape d'alimentation fluidique, et réitérées pour plusieurs instants de mesure $t_i$ successifs, jusqu'à ce qu'un critère de stabilité soit vérifié. A chaque itération i est ainsi associé un instant de mesure $t_i$, également appelé instant courant.

Lors d'une étape 200, on détermine, pour chaque site sensible de rang k allant de 1 à N, à l'instant courant $t_i$, un signal de mesure $S_k(t_i)$ représentatif des interactions entre les composés cibles et les récepteurs, à un instant de mesure $t_i$ pour ainsi obtenir un vecteur de mesure $\mathbf{S}(t_i)$.

Plus précisément, le capteur d'image acquiert, sur une durée $\Delta t$ séparant deux instants de mesure successifs $t_{i-1}$ et $t_i$, une pluralité d'images dites élémentaires $Ie_m$ de la matrice des N sites sensibles distincts, m étant le rang d'acquisition de l'image élémentaire Ie, à une fréquence d'échantillonnage $f_e$. La fréquence d'échantillonnage $f_e$ peut être de 10 images par seconde, et la durée $\Delta t$ d'acquisition peut être de quelques secondes, par exemple 4s.

Pour chaque image élémentaire $Ie_m$, l'unité de traitement détermine une valeur d'intensité optique élémentaire $(s_k)_m$ en faisant la moyenne de l'intensité optique $(s_k(i,j))_m$ acquise par chaque pixel i,j associé à un même site sensible de rang k, et en calcule une valeur moyenne $(\overline{s_k})_{\Delta t}$ sur la durée $\Delta t$ d'acquisition. Cette valeur moyenne $(\overline{s_k})_{\Delta t}$ correspond alors au signal optique de mesure $S_k(t_i)$, à l'instant courant $t_i$, associé au site sensible de rang k.

On obtient ainsi un vecteur de mesure $\mathbf{S}(t_i)$ à l'instant courant $t_i$, dont les coordonnées [$Si(ti)$, ..., $S_k(t_i)$, ... $S_N(t_i)$] sont les signaux de mesure des sites sensibles à l'instant courant $t_i$, de sorte que l'on peut écrire : $\mathbf{S}(t_i)=[S_k(t_i)]_{k=1,N}$.

Lors d'une étape 300, on calcule avantageusement un vecteur utile $\mathbf{Su}(t_i)$ à l'instant courant $t_i$, en soustrayant au vecteur de mesure $\mathbf{S}(t_i)$ déterminé précédemment une valeur de référence acquise pour chaque site sensible k lors de la phase initiale P1, de sorte que $\mathbf{Su}(t_i)$ = $\mathbf{S}(t_i)$ - $\mathbf{S}(\Delta t_{ref})$ = $[S_k(t_i)]_{k=1,N}$ - $[S_k(\Delta t_{ref})]_{k=1,N}$. La période de référence $\Delta t_{ref}$ est égale par exemple à plusieurs fois la durée d'acquisition $\Delta t$ au cours de la phase initiale P1, et peut être une période qui précède directement la phase d'injection P2. Ainsi, l'information associée au seul gaz porteur, donnée par $\mathbf{S}(\Delta t_{ref})$, est soustraite aux informations contenues dans $\mathbf{S}(t_i)$, permettant de mettre en évidence l'information liée essentiellement aux interactions entre les composés cibles et les récepteurs.

Lors d'une étape 400, on calcule un vecteur normé $\mathbf{Sn}(t_i)$ à l'instant courant $t_i$, à partir du vecteur de mesure $\mathbf{S}(t_i)$ à l'instant courant $t_i$ et d'une norme du vecteur de mesure $\mathbf{S}(t_i)$ à l'instant courant $t_i$. De préférence, on calcule le rapport entre le vecteur utile $\mathbf{Su}(t_i)$ à l'instant courant $t_i$ et une norme $\|\mathbf{Su}(t_i)\|$ à l'instant courant $t_i$ de celui-ci.

Notons que, d'une manière générale, la norme d'ordre p d'un vecteur $\mathbf{S}$ de dimension N et de coordonnées $[S_k]_{k=1,N}$ est définie par la relation suivante : $\|\mathbf{S}\|_p$ = $(\Sigma_{k=1,N} |S_k|^p)^{1/p}$, avec p un nombre, entier ou décimal, positif non nul. Ainsi, la norme 1 correspond à la somme de la valeur absolue des coordonnées du vecteur $\mathbf{S}$, et la norme 2 correspond à la norme euclidienne.

Dans cet exemple, on utilise de préférence la norme euclidienne (norme 2). Ainsi, le vecteur normé $\mathbf{Sn}(t_i)$ à l'instant courant $t_i$ est calculé par la relation suivante : $\mathbf{Sn}(t_i) = \mathbf{Su}(t_i) / (\Sigma_{k=1,N} |Su_k(t_i)|^2)^{1/2}$.

De préférence, le vecteur utile $\mathbf{Su}(t_i)$ à l'instant courant $t_i$ peut avoir été traité, au préalable, pour obtenir un vecteur dit corrigé $\mathbf{Sc}(t_i)$ pour lequel le bruit a été au moins en partie filtré et/ou le rapport signal sur bruit augmenté. Ainsi, à titre d'exemples, le vecteur corrigé $\mathbf{Sc}(t_i)$ peut être obtenu en appliquant un filtre passe-bas classique au vecteur utile $\mathbf{Su}(t_i)$, voire, en variante, en calculant le cumul des instants de mesure précédents : $\mathbf{Sc}(t_i)$ = $\mathbf{Su}(t_i)$ + $\mathbf{Sc}(t_{i-1})$.

Comme décrit plus loin, il apparaît, de manière surprenante, que les sensorgrammes formés sur la base d'un tel vecteur normé $\mathbf{Sn}(t_i)$ présentent, durant la phase d'injection P2, une partie transitoire d'une durée particulièrement réduite, suivie d'une partie stationnaire. Notons que ces parties transitoire et stationnaire ne correspondent pas aux régimes d'assimilation et d'équilibre décrits précédemment, dans la mesure où la phase d'injection ne comporte pas d'équilibre entre les cinétiques d'adsorption et de désorption entre les composés cibles et les récepteurs. Quoi qu'il en soit, il est cependant possible d'effectuer une caractérisation des composés cibles à partir de la partie stationnaire des sensorgrammes $Sn_k(t_i)$.

Lors d'une étape 500, on calcule un critère de stabilité tel que, une fois atteint, le procédé de caractérisation procède ensuite à la caractérisation des composés cibles, c'est-à-dire qu'il fournit un motif d'interaction, sous la forme par exemple d'un histogramme ou d'un diagramme en radar, à partir des coordonnées du vecteur normé $\mathbf{Sn}(t_i)$ à l'instant courant $t_i$. Dans cet exemple, le critère de stabilité est vérifié lorsque le vecteur normé $\mathbf{Sn}(t_i)$ présente une stabilité temporelle suffisante lors de la phase d'injection P2.

Aussi, une première sous-étape 510, avantageuse, peut consister à identifier la phase d'injection P2 au cours de l'étape d'alimentation fluidique. Pour cela, une approche revient à déterminer un paramètre dit d'injection $P_{inj}(t_i)$ à l'instant courant $t_i$. Ce paramètre d'injection $P_{inj}(t_i)$ peut être défini comme étant égal au maximum de la variance $V_k$ des signaux utiles $Su_k(t_i)$ (ou des signaux corrigés $Sc_k(t_i)$) calculée à l'instant courant $t_i$ sur une fenêtre glissante $t_i-T_{inj}$. La période $T_{inj}$ de la fenêtre glissante est égale à plusieurs fois la durée d'acquisition $\Delta t$, par exemple est égale à $5 \times \Delta t$. Aussi, le paramètre d'injection s'écrit : $P_{inj}(t_i)$ = $\max\limits_{k=1,N} V_k\left(Su_k(t_i - T_{inj})\right)$. D'autres formulations du paramètre d'injection sont également possibles (moyenne d'une différence temporelle, etc...).

La phase d'injection peut être dite identifiée lorsque le paramètre d'injection $P_{inj}(t_i)$ est supérieur ou égal à une valeur seuil $P_{inj,th}$: $P_{inj}(t_i) \geq P_{inj,th}$. Cette valeur seuil $P_{inj,th}$ peut être prédéterminée, par exemple à partir d'une base de données préalablement remplie relative à différents composés cibles, ou peut être déterminée au cours du procédé de caractérisation. A ce titre, la valeur seuil $P_{inj,th}$ peut être déterminée au cours de la phase initiale P1 de l'étape d'alimentation, comme étant égale au produit d'un coefficient et du maximum des valeurs du paramètre d'injection $P_{inj}(t_i)$ sur une période d'apprentissage $T_{learn}$, par exemple, égale à 10 ou 20 fois $\Delta t$, au cours de la phase initiale P1. Le coefficient peut être égal à 1.02, 1.05, 1.10, entre autres. Ainsi, on calcule $P_{inj,th}$ = $1.05 \times \max\limits_{T_{learn}} P_{inj}(t_i)$. Ensuite, après cette période d'apprentissage $T_{learn}$, la valeur seuil seuil $P_{inj,th}$ est imposée et reste constante au cours de toute l'étape d'alimentation fluidique. Un réapprentissage peut alors être effectué à nouveau lorsque le paramètre d'injection repasse de manière durable sous le seuil. Cela permet d'avoir une référence avantageusement plus récente.

Ensuite, une deuxième sous-étape 520 peut consister à déterminer la stabilité temporelle du vecteur normé $\mathbf{Sn}(t_i)$ au cours de l'étape d'alimentation fluidique. Pour cela, une approche revient à déterminer un paramètre dit de stabilité $P_{st}(t_i)$ à l'instant courant $t_i$. Ce paramètre de stabilité $P_{st}(t_i)$ peut être défini comme étant égal au maximum de la variance $V_k$ des signaux normés $Sn_k(t_i)$ calculée à l'instant courant $t_i$ sur une fenêtre glissante $t_i$-$T_{st}$. La période $T_{st}$ de la fenêtre glissante peut être différente ou égale à la période $T_{inj}$, et peut être égale à plusieurs fois la durée d'acquisition $\Delta t$, par exemple est égale à $5 \times \Delta t$. Aussi, le paramètre de stabilité s'écrit :

$$P_{st}(t_i) = \max_{k=1,N} V_k\big(Sn_k(t_i - T_{st})\big).$$

La stabilité temporelle peut être dite suffisante lorsque le paramètre de stabilité $P_{st}(t_i)$ est inférieure ou égale à une valeur seuil $P_{st,th}$ : $P_{st}(t_i) \leq P_{st,th}$. Cette valeur seuil $P_{st,th}$ peut être prédéterminée, par exemple à partir d'une base de données préalablement remplie relative à différents composés cibles, ou peut être déterminée au cours du procédé de caractérisation. A ce titre, la valeur seuil $P_{st,th}$ peut être déterminée en temps réel au cours de l'étape d'alimentation, comme étant égale au produit d'un coefficient et du maximum des valeurs du paramètre de stabilité $P_{st}(t_i)$ sur la période d'apprentissage $T_{learn}$ au cours de la phase initiale P1. Le coefficient peut être égal à 1.02, 1.05, 1.10, entre autres. Ainsi, on calcule $$P_{st,th} = 1.05 \times \max_{T_{learn}} P_{st}(t_i).$$ Ensuite, après cette période d'apprentissage $T_{learn}$, la valeur seuil seuil $P_{st,th}$ reste constante au cours de toute l'étape d'alimentation fluidique.

Dans le cas où une seule condition ou aucune condition n'est atteinte, le critère de stabilité n'est pas vérifié et le procédé réitère les étapes 200 à 500 d'acquisition des images et de traitement des données. Ainsi, dans le cas où $P_{inj}(t_i) < P_{inj,th}$, on considère que l'étape d'alimentation est encore dans la phase initiale P1 de l'étape d'alimentation fluidique, et donc que l'échantillon gazeux ne contient que le gaz porteur et non pas encore les composés cibles. Dans le cas où $P_{st}(t_i) > P_{st,th}$, on considère que l'étape d'alimentation est dans la phase d'injection P2 de l'étape d'alimentation fluidique et donc que l'échantillon gazeux contient les composés cibles, mais que la stabilité temporelle du vecteur normé $\mathbf{Sn}(t_i)$ n'est pas suffisante pour permettre la caractérisation des composés cibles. En revanche, le critère de stabilité est dit atteint lorsque les deux conditions ci-dessus sont réunies, à savoir lorsque $P_{inj}(t_i) \geq P_{inj,th}$ et lorsque $P_{st}(t_i) \leq P_{st,th}$. On considère alors que le vecteur normé $\mathbf{Sn}(t_i)$ présente une stabilité suffisante pour permettre la caractérisation des composés cibles alors présents dans l'échantillon gazeux, et le procédé passe à l'étape suivante de fourniture du motif d'interaction.

Lors d'une étape 600, on caractérise les composés cibles à partir du vecteur normé $\mathbf{Sn}(t_i)$ à l'instant courant $t_i$. Il s'agit alors de fournir une représentation sous forme d'un histogramme, d'un diagramme en radar, ou autre, des coordonnées $Sn_k(t_i)$ du vecteur normé à l'instant courant $t_i$ correspondant à l'instant de mesure final. Avantageusement, le procédé de caractérisation des composés cibles permet de fournir, en plus du motif d'interaction, des informations complémentaires telles que la quantité totale d'interactions entre les composés cibles et les récepteurs au cours de la phase d'injection P2, ainsi que l'évolution temporelle d'une intensité d'interaction. A ce titre, l'intensité d'interaction $I_{int}(t_i)$ à l'instant courant $t_i$ correspond par exemple à la norme euclidienne (norme 2) du vecteur normé $\mathbf{Sn}(t_i)$, et la quantité totale d'interaction correspond à l'intégrale sur la phase d'injection P2, de l'intensité d'interaction $I_{int}(t_i)$.

Ainsi, comme détaillé plus loin, le procédé de caractérisation permet de fournir un motif d'interaction des composés cibles avec les récepteurs des sites sensibles, de manière plus rapide et plus simple que dans le cas de l'exemple de l'art antérieur mentionné précédemment. En effet, il apparaît que le vecteur normé $\mathbf{Sn}(t_i)$ présente une partie stationnaire lors de la phase d'injection P2 des composés cibles. Le motif d'interaction peut donc être obtenu à partir du vecteur normé $\mathbf{Sn}(t_i)$ dès que le critère de stabilité montre que la partie stationnaire est atteinte. Il apparaît à ce titre que cette partie stationnaire est précédée d'une partie transitoire très courte, qui traduit le début de la phase d'injection P2. Il est alors possible de fournir le motif d'interaction dans un délai réduit, bien plus court que dans le cas de l'art antérieur dans la mesure où il n'est plus nécessaire d'attendre que s'établisse le régime stationnaire d'équilibre P2.2.

De plus, le procédé de caractérisation peut fournir le motif d'interaction alors même que la concentration $c_A(t)$ des composés cibles dans la chambre de mesure n'est pas constante, et donc alors même que les sensorgrammes ne montrent pas le régime stationnaire d'équilibre P2.2. Il est alors possible de caractériser les composés cibles en « conditions réelles », c'est-à-dire avec des conditions opératoires et un protocole d'alimentation fluidique simplifiés. On simplifie ainsi le procédé de caractérisation, et on réduit également la complexité structurelle du dispositif d'alimentation fluidique, en termes notamment de vannes et de contrôleur d'écoulement massique. Simultanément, cet indicateur normé donne accès à une vue sur la compétition qui pourrait s'établir dans la cinétique inter-sites et être une caractéristique des composés cibles étudiés.

Les figures 4A et 4B illustrent la sous-étape 510 d'identification de la phase d'injection P2. La fig.4A montre l'évolution temporelle des signaux utiles $Su_k(t_i)$, ainsi que l'évolution temporelle du paramètre d'injection $P_{inj}(t_i)$ et celle de sa valeur seuil $P_{inj,th}(t_i)$, dans le cas de sensorgrammes à profils de type dégradé similaires à ceux de la fig.2B. La fig.4B montre une partie en détail de la fig.4A. La fig.4B illustre en particulier, en trait pointillé, l'évolution temporelle du paramètre d'injection $P_{inj}(t_i)$, et en trait continu, celle de la valeur seuil $P_{inj,th}(t_i)$. il apparaît que la valeur seuil $P_{inj,th}(t_i)$ est dans une phase d'apprentissage lors de la phase initiale P1 de l'étape d'alimentation fluidique, et prend ensuite une valeur imposée et constante. Le paramètre d'injection $P_{inj}(t_i)$ permet, au cours de la période $T_{learn}$, de définir la valeur finale imposée de $P_{inj,th}$. Ensuite, son évolution temporelle permet d'identifier la phase d'injection P2, puis celle de la phase de dissociation P3, selon que $P_{inj}(t_i)$ est supérieur ou égal à $P_{inj,th}$ ou en est inférieur.

Les figures 5A et 5B illustrent un exemple d'évolution temporelle (fig.5A) du vecteur corrigé $\mathbf{Sc}(t_i)$ calculé à partir du vecteur utile $\mathbf{Su}(t_i)$, et un exemple d'évolution temporelle (fig.5B) du vecteur normé $\mathbf{Sn}(t_i)$ calculé à partir du vecteur corrigé $\mathbf{Sc}(t_i)$, ainsi que celle du paramètre de stabilité $P_{st}(t_i)$. Ces vecteurs ont également été obtenus à partir de sensorgrammes à profil de type dégradé. Le vecteur corrigé $\mathbf{Sc}(t_i)$ est calculé à partir du cumul des valeurs du vecteur utile : $\mathbf{Sc}(t_i) = \mathbf{Su}(t_i) + \mathbf{Sc}(t_{i-1})$. Cette approche permet de fournir un vecteur corrigé présentant un rapport signal sur bruit plus important. La paramètre de stabilité $P_{st}(t_i)$ montre une première partie avec une valeur quasi nulle lors de la phase initiale P1, suivie d'une partie transitoire avec une valeur importante au début de la phase d'injection P2, puis d'une partie stationnaire avec une valeur quasi nulle dans la phase d'injection P2. Cette partie stationnaire est alors mise à profit pour caractériser les composés cibles. Il apparaît que la durée de la phase transitoire est particulièrement réduite, et bien inférieure à la durée du régime transitoire d'assimilation P2.1 dans le cas des sensorgrammes à profil de type conventionnel. Il est alors possible de caractériser les composés cibles bien plus rapidement que dans le cas des procédés de caractérisation conventionnels, qu'il y ait ou non l'établissement d'un équilibre P2.2 entre les cinétiques d'absorption et de désorption.

Les figures 6A et 6B illustrent un autre exemple d'évolution temporelle (fig.6A) du vecteur corrigé $\mathbf{Sc}(t_i)$ calculé à partir du vecteur utile $\mathbf{Su}(t_i)$, et un exemple d'évolution temporelle (fig.6B) du vecteur normé $\mathbf{Sn}(t_i)$ calculé à partir du vecteur corrigé $\mathbf{Sc}(t_i)$, ainsi que celle du paramètre de stabilité $P_{st}(t_i)$. Ces vecteurs ont également été obtenus à partir de sensorgrammes à profil de type dégradé. Le vecteur corrigé $\mathbf{Sc}(t_i)$ est calculé ici en appliquant un filtrage passe-bas classique au vecteur utile $\mathbf{Su}(t_i)$. Cette approche permet de réduire le bruit présent dans le vecteur utile $\mathbf{Su}(t_i)$. Le paramètre de stabilité $P_{st}(t_i)$ montre une première partie avec de fortes variations lors de la phase initiale P1, suivie d'une partie transitoire avec une valeur importante au début de la phase d'injection P2, puis d'une partie stationnaire avec une valeur quasi nulle dans la phase d'injection P2. Cette partie stationnaire est ici également mise à profit pour caractériser les composés cibles. Comme précédemment, il est possible de caractériser les composés cibles bien plus rapidement que dans le cas des procédés de caractérisation conventionnels, qu'il y ait ou non l'établissement d'un équilibre entre les cinétiques d'absorption et de désorption.

Les figures 7A et 7B illustrent des exemples d'informations fournies lors de l'étape de caractérisation des composés cibles. Un motif d'interaction peut alors être fourni (fig.7A), ici sous la forme d'un diagramme en radar indiquant la coordonnée $Sn_k(t_i)$ pour chaque site sensible de rang k allant de 1 à 45, à l'instant de mesure final $t_i$. De plus, l'évolution temporelle de l'intensité d'interaction $I_{int}(t_i)$ peut également être fournie (fig.7B). Elle correspond ici au calcul de la norme euclidienne du vecteur normé $\mathbf{Sn}(t_i)$ à l'instant courant $t_i$. Elle renseigne ainsi l'utilisateur sur le peuplement instantané des composés cibles au sein des récepteurs au cours du temps, ce qui peut être particulièrement important dans certains domaines, comme celui de la santé. A noter que l'intégrale de l'intensité d'interaction $I_{int}(t_i)$ sur la période de la phase d'injection P2 permet d'en déduire un niveau d'exposition des récepteurs aux composés cibles.

Des informations supplémentaires peuvent également être déduites du vecteur normé $\mathbf{Sn}(t_i)$. Ainsi, certaines coordonnées $Sn_k(t_i)$ peuvent montrer un gradient stationnaire (pente sensiblement constante), lors de la phase d'injection P2. La valeur de la pente peut également participer à caractériser les composés cibles. Ces paramètres peuvent être déterminés sur une période donnée de la phase d'injection P2.

Des modes de réalisation particuliers viennent d'être décrits. Différentes variantes et modifications apparaîtront à l'homme du métier.

Comme mentionné précédemment, l'échantillon fluide contenant les composés cibles peut être gazeux ou liquide. Le procédé de caractérisation est de préférence mis en œuvre par un nez électronique à imagerie SPR avec des échantillons gazeux, mais d'autres technologies d'analyse peuvent être mises en œuvre, telles que l'analyse par résonateurs électromécaniques de type NEMS ou MEMS.

## Revendications

1. Procédé de caractérisation de composés cibles, par un système d'analyse comportant une chambre de mesure destinée à recevoir les composés cibles contenus dans un échantillon fluide, dans laquelle est située une pluralité de sites sensibles distincts comportant chacun des récepteurs aptes à interagir avec les composés cibles, le procédé comportant les étapes suivantes :

    o alimentation fluidique (100) d'un échantillon fluide dans la chambre de mesure, comportant une phase d'injection $P_2$ dans laquelle l'échantillon fluide est formé d'un fluide porteur et desdits composés cibles ;
    o détermination (200), lors de l'étape d'alimentation, à un instant de mesure $t_i$, pour chaque site sensible, d'un signal de mesure $S_k(t_i)$ représentatif des interactions entre les composés cibles et les récepteurs, k étant le rang du site sensible considéré, de manière à obtenir un vecteur dit de mesure $S(t_i)$ formé des signaux de mesure $S_k(t_i)$ acquis à l'instant de mesure $t_i$; et
o calcul (400), à l'instant de mesure $t_i$, d'un vecteur normé $\mathbf{Sn}(t_i)$ à partir du vecteur de mesure $S(t_i)$ à l'instant de mesure $t_i$ et d'une norme $\|S(t_i)\|$ calculée à partir du vecteur de mesure $S(t_i)$ à l'instant de mesure $t_i$ ;

**caractérisé en ce qu'**il comporte en outre l'étape suivante :

o réitération (500) des étapes de détermination des signaux de mesure et de calcul du vecteur normé, en incrémentant l'instant de mesure, jusqu'à l'atteinte d'un critère de stabilité, de manière à obtenir une caractérisation (600) des composés cibles à partir du vecteur normé $\mathbf{Sn}(t_i)$ à l'instant de mesure $t_i$.

2. Procédé selon la revendication 1, dans lequel :

o l'étape d'alimentation fluidique comporte, au préalable de la phase d'injection P2, une phase initiale $P_1$ dans laquelle l'échantillon fluide est formé du fluide porteur sans lesdits composés cibles ;
o l'étape de détermination (200) du signal de mesure $S_k(t_i)$ comporte un calcul d'un vecteur dit utile $\mathbf{Su}(t_i)$ à l'instant de mesure $t_i$, par soustraction au vecteur de mesure $S(t_i)$ d'un vecteur dit de référence $S(\Delta t_{ref})$ déterminé lors de la phase initiale P1 pour une période de mesure $\Delta t_{ref}$ prédéterminée ;
o le vecteur normé $\mathbf{Sn}(t_i)$ étant calculé à partir du vecteur utile $\mathbf{Su}(t_i)$.

3. Procédé selon la revendication 1 ou 2, dans lequel l'étape de détermination (200) du signal de mesure $S_k(t_i)$ comporte un calcul d'un vecteur dit corrigé $\mathbf{Sc}(t_i)$ à partir du vecteur de mesure $S(t_i)$ par application d'un filtre passe-bas ou d'un cumul des valeurs du vecteur de mesure $S(t_i)$ aux instants de mesure précédents.

4. Procédé selon l'une quelconque des revendications 1 à 3, dans lequel le critère de stabilité comporte une comparaison, à l'instant de mesure $t_i$, d'un paramètre dit de stabilité $P_{st}(t_i)$ calculé à partir des coordonnées $Sn_k(t_i)$ du vecteur normé $\mathbf{Sn}(t_i)$ sur une fenêtre glissante $t_i\text{-}T_{st}$, vis-à-vis d'une valeur seuil déterminée $P_{st,th}$.

5. Procédé selon la revendication 4, dans lequel le paramètre de stabilité $P_{st}(t_i)$ est le maximum parmi des variances calculées à l'instant de mesure $t_i$ pour les coordonnées $Sn_k(t_i\text{-}T_{st})$ du vecteur normé $\mathbf{Sn}$ sur une fenêtre glissante $t_i\text{-}T_{st}$.

6. Procédé selon l'une quelconque des revendications 1 à 5, dans lequel le critère de stabilité comporte une comparaison, à l'instant de mesure $t_i$, d'un paramètre dit d'injection $P_{inj}(t_i)$ calculé à partir des coordonnées $S_k(t_i)$ du vecteur de mesure $S(t_i)$ sur une fenêtre glissante $t_i\text{-}T_{inj}$, vis-à-vis d'une valeur seuil déterminée $P_{inj,th}$.

7. Procédé selon la revendication 6, dans lequel le paramètre d'injection $P_{inj}(t_i)$ est le maximum parmi des variances calculées à l'instant de mesure $t_i$ pour les coordonnées $S_k(t_i\text{-}T_{inj})$ du vecteur de mesure $S$ sur une fenêtre glissante $t_i\text{-}T_{inj}$.

8. Procédé selon l'une quelconque des revendications 1 à 7, dans lequel la norme $\|S(t_i)\|$ est la norme euclidienne.

9. Procédé selon l'une quelconque des revendications 1 à 8, dans lequel l'étape de caractérisation (600) comporte la fourniture d'au moins un paramètre caractéristique d'une évolution temporelle de la norme euclidienne du vecteur normé $\mathbf{Sn}$ lors de la phase d'injection P2.

10. Procédé selon la revendication 9, dans lequel l'étape de caractérisation (600) comporte un calcul d'une intégrale, sur la durée de la phase d'injection P2, de la norme euclidienne du vecteur normé $\mathbf{Sn}$.

11. Procédé selon l'une quelconque des revendications 1 à 10, dans lequel le système d'analyse est un nez électronique à imagerie par résonance plasmonique de surface, ou est un système d'analyse comportant une pluralité de résonateurs électromécaniques distincts formant chacun un site sensible.

**Patentansprüche**

1. Verfahren zur Charakterisierung von Zielverbindungen durch ein Analysesystem, das eine zum Empfang der in einer Fluidprobe enthaltenen Zielverbindungen bestimmte Messkammer aufweist, in der sich eine Vielzahl unterschiedlicher empfindlicher Stellen befindet, die je Rezeptoren aufweisen, die geeignet sind, mit den Zielverbindungen zu interagieren, wobei das Verfahren die folgenden Schritte aufweist:

o Fluideinspeisung (100) einer Fluidprobe in die Messkammer, die eine Injektionsphase P2 aufweist, in der die Fluidprobe von einem Trägerfluid und von den Zielverbindungen geformt wird;

o Bestimmung (200), im Einspeisungsschritt, zu einem Messzeitpunkt $t_i$, für jede empfindliche Stelle, eines für die Interaktionen zwischen den Zielverbindungen und den Rezeptoren repräsentativen Messsignals $S_k(t_i)$, wobei k der Rang der betrachteten empfindlichen Stelle ist, um einen so genannten Messvektor $S(t_i)$ zu erhalten, der von den zum Messzeitpunkt $t_i$ erfassten Messsignalen $S_k(t_i)$ geformt wird; und

o Berechnung (400), zum Messzeitpunkt $t_i$, eines normierten Vektors $Sn(t_i)$ ausgehend vom Messvektor $S(t_i)$ zum Messzeitpunkt $t_i$ und einer Norm $|S(t_i)|$ berechnet ausgehend vom Messvektor $S(t_i)$ zum Messzeitpunkt $t_i$;

**dadurch gekennzeichnet, dass** es außerdem den folgenden Schritt aufweist:

o Wiederholung (500) der Schritte der Bestimmung der Messsignale und der Berechnung des normierten Vektors durch Inkrementieren des Messzeitpunkts bis zum Erreichen eines Stabilitätskriteriums, um eine Charakterisierung (600) der Zielverbindungen ausgehend vom normierten Vektor $Sn(t_i)$ zum Messzeitpunkt $t_i$ zu erhalten.

2. Verfahren nach Anspruch 1, wobei:

o der Schritt der Fluideinspeisung vor der Injektionsphase P2 eine Anfangsphase P1 aufweist, in der die Fluidprobe vom Trägerfluid ohne die Zielverbindungen geformt wird;

o der Schritt der Bestimmung (200) des Messsignals $S_k(t_i)$ eine Berechnung eines so genannten Nutzvektors $Su(t_i)$ zum Messzeitpunkt $t_i$ durch Subtraktion vom Messvektor $S(t_i)$ eines so genannten Bezugsvektors $S(\Delta t_{ref})$ enthält, der in der Anfangsphase P1 für eine vorbestimmte Messperiode $\Delta t_{ref}$ bestimmt wird;

o der normierte Vektor $Sn(t_i)$ ausgehend vom Nutzvektor $Su(t_i)$ berechnet wird.

3. Verfahren nach Anspruch 1 oder 2, wobei der Schritt der Bestimmung (200) des Messsignals $S_k(t_i)$ eine Berechnung eines so genannten korrigierten Vektors $Sc(t_i)$ ausgehend vom Messvektor $S(t_i)$ durch Anwendung eines Tiefpassfilters oder einer Kumulierung der Werte des Messvektors $S(t_i)$ zu den vorhergehenden Messzeitpunkten aufweist.

4. Verfahren nach einem der Ansprüche 1 bis 3, wobei das Stabilitätskriterium einen Vergleich, zum Messzeitpunkt $t_i$, eines ausgehend von den Koordinaten $Sn_k(t_i)$ des normierten Vektors $Sn(t_i)$ berechneten so genannten Stabilitätsparameters $P_{st}(t_i)$ in einem

gleitenden Fenster $t_i$-$T_{st}$ gegenüber einem bestimmten Schwellwert $P_{st,th}$ aufweist.

5. Verfahren nach Anspruch 4, wobei der Stabilitätsparameter $P_{st}(t_i)$ das Maximum unter zum Messzeitpunkt $t_i$ für die Koordinaten $Sn_k(t_i$-$T_{st})$ des normierten Vektors Sn in einem gleitenden Fenster $t_i$-$T_{st}$ berechneten Varianzen ist.

6. Verfahren nach einem der Ansprüche 1 bis 5, wobei das Stabilitätskriterium einen Vergleich zum Messzeitpunkt $t_i$ eines ausgehend von den Koordinaten $S_k(t_i)$ des Messvektors $S(t_i)$ in einem gleitenden Fenster $t_i$-$T_{inj}$ berechneten so genannten Injektionsparameters $P_{inj}(t_i)$ gegenüber einem bestimmten Schwellwert $P_{inj,th}$ aufweist.

7. Verfahren nach Anspruch 6, wobei der Injektionsparameter $P_{inj}(t_i)$ das Maximum unter zum Messzeitpunkt $t_i$ für die Koordinaten $S_k(t_i$-$T_{inj})$ des Messvektors S in einem gleitenden Fenster $t_i$-$T_{inj}$ berechneten Varianzen ist.

8. Verfahren nach einem der Ansprüche 1 bis 7, wobei die Norm $|S(t_i)|$ die euklidische Norm ist.

9. Verfahren nach einem der Ansprüche 1 bis 8, wobei der Charakterisierungsschritt (600) die Bereitstellung mindestens eines charakteristischen Parameters einer zeitlichen Entwicklung der euklidischen Norm des normierten Vektors Sn in der Injektionsphase P2 aufweist.

10. Verfahren nach Anspruch 9, wobei der Charakterisierungsschritt (600) eine Berechnung eines Integrals der euklidischen Norm des normierten Vektors Sn über die Dauer der Injektionsphase P2 aufweist.

11. Verfahren nach einem der Ansprüche 1 bis 10, wobei das Analysesystem eine elektronische Nase mit Bildgebung durch Oberflächenplasmonenresonanz oder ein Analysesystem ist, das eine Vielzahl unterschiedlicher elektromechanischer Resonatoren aufweist, die je eine empfindliche Stelle bilden.

**Claims**

1. A method for characterizing target compounds, with an analyzing system comprising a measurement chamber intended to receive target compounds contained in a fluid sample, in which measurement chamber are located a plurality of distinct sensitive sites each comprising receptors that are able to interact with the target compounds, the method comprising the following steps:

• fluidically supplying (100) a fluid sample to the measurement chamber, this comprising an injecting phase $P_2$ in which the fluid sample is formed from a carrier fluid and said target compounds;

• determining (200), in the supplying step, at a measurement time $t_i$, for each sensitive site, a measurement signal $S_k(t_i)$ representative of the interactions between the target compounds and the receptors, k being the rank of the sensitive site in question, so as to obtain a measurement vector $S(t_i)$ formed from the measurement signals $S_k(t_i)$ acquired at the measurement time $t_i$; and

• computing (400), at the measurement time $t_i$, a normalized vector $Sn(t_i)$ from the measurement vector $S(t_i)$ at the measurement time $t_i$, and from a norm $\|S(t_i)\|$ computed from the measurement vector $S(t_i)$ at the measurement time $t_i$;

**characterized in that** it furthermore comprises the following step:

• reiterating (500) the steps of determining measurement signals and of computing the normalized vector, while incrementing the measurement time, until a stability criterion is met, so as to obtain a characterization (600) of the target compounds on the basis of the normalized vector $Sn(t_i)$ at the measurement time $t_i$.

2. The method as claimed in claim 1, wherein:

• the fluid-supplying step comprises, prior to the injecting phase P2, an initial phase $P_1$ in which the fluid sample is formed from the carrier fluid without said target compounds;
• the step of determining (200) the measurement signal $S_k(t_i)$ comprises computing a useful vector $Su(t_i)$, at the measurement time $t_i$, by subtracting from the measurement vector $S(t_i)$ a reference vector $S(\Delta t_{ref})$ determined in the initial phase P1 in a predetermined measurement period $\Delta t_{ref}$;
• the normalized vector $\mathbf{Sn}(t_i)$ being computed from the useful vector $\mathbf{Su}(t_i)$.

3. The method as claimed in claim 1 or 2, wherein the step of determining (200) the measurement signal $S_k(t_i)$ comprises computing a corrected vector $Sc(t_i)$ from the measurement vector $S(t_i)$ with application of a low-pass filter or from a sum of the values of the measurement vector $\mathbf{S}(t_i)$ at the previous measurement times.

4. The method as claimed in any one of claims 1 to 3, wherein the stability criterion comprises a comparison, at the measurement time $t_i$, of a stability parameter $P_{st}(t_i)$ computed from the coordinates $Sn_k(t_i)$ of the normalized vector $\mathbf{Sn}(t_i)$ in a moving window $t_i$-$T_{st}$, to a determined threshold value $P_{st,th}$.

5. The method as claimed in claim 4, wherein the stability parameter $P_{st}(t_i)$ is the maximum among the variances computed at the measurement time $t_i$ for the coordinates $Sn_k(t_i$-$T_{st})$ of the normalized vector Sn in a moving window $t_i$-$T_{st}$.

6. The method as claimed in any one of claims 1 to 5, wherein the stability criterion comprises a comparison, at the measurement time $t_i$, of a injection parameter $P_{inj}(t_i)$ computed from the coordinates $S_k(t_i)$ of the measurement vector $\mathbf{S}(t_i)$ in a moving window $t_i$-$T_{inj}$, to a determined threshold value $P_{inj,th}$.

7. The method as claimed in claim 6, wherein the injection parameter $P_{inj}(t_i)$ is the maximum among the variances computed at the measurement time $t_i$ for the coordinates $S_k(t_i$-$T_{inj})$ of the measurement vector S in a moving window $t_i$-$T_{inj}$.

8. The method as claimed in any one of claims 1 to 7, wherein the norm $\|\mathbf{S}(t_i)\|$ is the Euclidean norm.

9. The method as claimed in any one of claims 1 to 8, wherein the characterizing step (600) comprises providing at least one parameter characteristic of a variation as a function of time in the Euclidean norm of the normalized vector Sn in the injecting phase P2.

10. The method as claimed in claim 9, wherein the characterizing step (600) comprises computing an integral, over the duration of the injecting phase P2, of the Euclidean norm of the normalized vector **Sn.**

11. The method as claimed in any one of claims 1 to 10, wherein the analyzing system is an electronic nose based on surface-plasmon-resonance imaging, or is an analyzing system comprising a plurality of distinct electromechanical resonators each forming one sensitive site.

1

2

5

4

3

$\theta_R$

8

7

**Fig.1A**

6

5

**Fig.1B**

**Fig.2A**

**Fig.2B**

100

200     $\mathbf{S}(t_i) = [S_k(t_i)]_{k=1,N}$

300     $\mathbf{Su}(t_i) = \mathbf{S}(t_i) - \mathbf{S}(\Delta t_{ref})$

400     $\mathbf{Sn}(t_i) = \mathbf{Su}(t_i) / \|\mathbf{Su}(t_i)\|_p$

$i := i+1$     500     $P_{inj}(t_i) \geq P_{inj,th}$
$P_{st}(t_i) \leq P_{st,th}$

600     $\mathbf{Sn}(t_i) \; ; \; I_{int}(t_i)$

**Fig.3**

**Fig.4A**

**Fig.4B**

**Fig.5A**

**Fig.5B**

**Fig.6A**

**Fig.6B**

**Fig.7A**

**Fig.7B**

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

**Documents brevets cités dans la description**

- WO 2018158458 A

- EP 3184485 A

**Littérature non-brevet citée dans la description**

- **BRENET et al.** Highly-Selective Optoelectronic Nose based on Surface Plasmon Resonance Imaging for Sensing Gas Phase Volatile Organic Compounds. *Anal. Chem.,* 2018, vol. 90 (16), 9879-9887
- Odour classification system for continuous monitoring applications. **TRINCAVELLI M et al.** SENSORS AND ACTUATORS B: CHEMICAL. ELSEVIER BV, 20 Mars 2009, vol. 139, 265-273

- **PEARCE T C.** Computational parallels between the biological olfactory pathway and its analogue 'The Electronic Nose': Part II. Sensor-based machine olfaction. *BIOSYSTEMS, NORTH-HOLLAND, AMSTERDAM, NL,* 1997, vol. 41, 69-90
- **HAN FAN et al.** A cluster analysis approach based on exploiting density peaks for gas discrimination with electronic noses in open environments. *SENSORS AND ACTUATORS B: CHEMICAL,* 02 Décembre 2017, vol. 259, 183-203